(19) Europäisches Patentamt
European Patent Office
Office européen des brevets

(11) **EP 3 423 823 B1**

(12) **FASCICULE DE BREVET EUROPEEN**

(45) Date de publication et mention
de la délivrance du brevet:
**30.08.2023 Bulletin 2023/35**

(21) Numéro de dépôt: **17708783.0**

(22) Date de dépôt: **03.03.2017**

(51) Classification Internationale des Brevets (IPC):
**G01N 25/02** *(2006.01)*   **G01N 21/84** *(2006.01)*
**G01N 33/02** *(2006.01)*   **G01N 21/47** *(2006.01)*
**G01N 21/49** *(2006.01)*

(52) Classification Coopérative des Brevets (CPC):
**G01N 33/02; G01N 21/4738; G01N 25/02;**
G01N 21/49; G01N 2021/8405

(86) Numéro de dépôt international:
**PCT/EP2017/055097**

(87) Numéro de publication internationale:
**WO 2017/149151 (08.09.2017 Gazette 2017/36)**

(54) **DISPOSITIF ET PROCÉDÉ D'ANALYSE D'UN CHANGEMENT D'ÉTAT PHYSIQUE D'UNE MATIÈRE NON PUREMENT GAZEUSE**

VORRICHTUNG UND VERFAHREN ZUR ANALYSE EINER PHYSIKALISCHEN ZUSTANDSÄNDERUNG EINES NICHT REIN GASFÖRMIGEN STOFFES

DEVICE AND PROCESS FOR ANALYSING A CHANGE IN PHYSICAL STATE OF A MATTER THAT IS NOT PURELY GASEOUS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.03.2016 FR 1651843**

(43) Date de publication de la demande:
**09.01.2019 Bulletin 2019/02**

(73) Titulaire: **Formulaction**
**31200 Toulouse (FR)**

(72) Inventeur: **BRAMBILLA, Giovanni**
**31590 Saint Marcel Paulel (FR)**

(74) Mandataire: **BARRE LAFORGUE**
**35, rue Lancefoc**
**31000 Toulouse (FR)**

(56) Documents cités:
**US-A1- 2005 037 499**

- **TIPPETTS M ET AL: "Effect of cooling rate on lipid crystallization in oil-in-water emulsions", FOOD RESEARCH INTERNATIONAL, ELSEVIER APPLIED SCIENCE, BARKING, GB, vol. 42, no. 7, 1 août 2009 (2009-08-01), pages 847-855, XP026131997, ISSN: 0963-9969, DOI: 10.1016/J.FOODRES.2009.03.009 [extrait le 2009-03-25]**
- **DA COSTA GERMÁN: "Large scale image projection setup for observation of flocculation in heavy oil/water emulsions", REVIEW OF SCIENTIFIC INSTRUMENTS, AIP, MELVILLE, NY, US, vol. 76, no. 9, 16 septembre 2005 (2005-09-16), pages 96105-096105, XP012079759, ISSN: 0034-6748**
- **PIZZINO A ET AL: "On-line light backscattering tracking of the transitional phase inversion of emulsions", COLLOIDS AND SURFACES. A, PHYSICACHEMICAL AND ENGINEERING ASPECTS, ELSEVIER, AMSTERDAM, NL, vol. 338, no. 1-3, 15 avril 2009 (2009-04-15), pages 148-154, XP025959074, ISSN: 0927-7757, DOI: 10.1016/J.COLSURFA.2008.05.041 [extrait le 2008-06-03]**

- CHALLIS R E ET AL: "Ultrasound techniques for characterizing colloidal dispersions; Ultrasound techniques for characterizing colloidal dispersions", REPORTS ON PROGRESS IN PHYSICS, INSTITUTE OF PHYSICS PUBLISHING, BRISTOL, GB, vol. 68, no. 7, 17 juin 2005 (2005-06-17), pages 1541-1637, XP020084659, ISSN: 0034-4885, DOI: 10.1088/0034-4885/68/7/R01
- Hampton: "Sitting Drop Vapor Diffusion Crystallization", , 31 December 2001 (2001-12-31), XP055468184, Hampton Research Corporation Retrieved from the Internet: URL:http://www.img.bio.uni-goettingen.de/m s-www/internal/methods/Crystalscreens/CG10 1SDC.pdf [retrieved on 2018-04-18]
- Karsten Dierks ET AL: "Dynamic Light Scattering in Protein Crystallization Droplets: Adaptations for Analysis and Optimization of Crystallization Processes", Crystal Growth & Design, vol. 8, no. 5, 1 May 2008 (2008-05-01), pages 1628-1634, XP055186833, ISSN: 1528-7483, DOI: 10.1021/cg701067r
- GIOVENZANA VALENTINA ET AL: "Assessment of Tempering Degree during the Chocolate Pre-Crystallisation Phase Using near Infrared Spectroscopy", NIR NEWS, [Online] vol. 26, no. 1, 1 February 2015 (2015-02-01), pages 8-10, XP055935395, GB ISSN: 0960-3360, DOI: 10.1255/nirn.1501 Retrieved from the Internet: URL:http://journals.sagepub.com/doi/pdf/10 .12 55/nirn.1501> [retrieved on 2022-06-24]
- LIBERA M ET AL: "TIME-RESOLVED REFLECTION AND TRANSMISSION STUDIES OF AMORPHOUS GE-TE THIN-FILM CRYSTALLIZATION", JOURNAL OF APPLIED PHYSICS, AMERICAN INSTITUTE OF PHYSICS, 2 HUNTINGTON QUADRANGLE, MELVILLE, NY 11747, vol. 73, no. 5, 1 March 1993 (1993-03-01), pages 2272-2282, XP000358723, ISSN: 0021-8979, DOI: 10.1063/1.353132

**Description**

L'invention concerne un dispositif et un procédé d'analyse d'un changement d'état physique d'une matière non purement gazeuse selon les revendications 1 et 6, respectivement

L'invention concerne également l'utilisation d'un tel dispositif pour évaluer un risque de survenue d'un blanchiment de chocolat à toute étape de son élaboration selon la revendication 13.

L'invention est aussi établie dans les revendications dépendantes 2 à 5 et 7 à 12.

Dans tout le texte, l'expression « changement d'état physique d'une matière » désigne tout changement -notamment toute transitiond'état physique d'une matière non purement gazeuse choisie dans le groupe formé d'un changement liquide/solide -notamment d'une cristallisation ou d'une transition liquide/solide amorphe (transition vitreuse) ou d'une gélification (transition sol/gel)- et d'un changement solide/liquide (ou fusion) d'une matière solide (amorphe ou cristalline ou sous forme d'un gel) ou un changement solide/solide, notamment entre deux états cristallins -transition cristalline ou transition polymorphique- ou entre un état cristallin et un état amorphe solide, intervenant dans une matière non purement gazeuse. Cette expression englobe en particulier des changements d'état physique d'une matière non purement gazeuse qui s'accompagnent d'une modification de la structure chimique de cette matière non purement gazeuse. Il peut s'agir d'une dénaturation -par exemple, une dénaturation de protéines-, d'une réticulation, d'une polymérisation, etc.

On connait des dispositifs et des méthodes d'analyse d'une transition de phase d'une matière, par exemple la DSC (« *Differential Scanning Calorimetry* »)*,* la DTA (« *Differential Thermal Analysis* ») ou la thermogravimétrie. De telles analyses sont nécessairement mises en oeuvre sur des échantillons dont la taille n'est pas suffisamment représentative de la totalité de la matière à analyser. Elles nécessitent en outre des manipulations fines de préparation et de conditionnement des échantillons qui ne sont souvent pas compatibles avec une mise en oeuvre à l'échelle industrielle. En outre, de telles manipulations en vue du conditionnement des échantillons sont de nature à modifier les propriétés de la matière à analyser et par conséquent à limiter la pertinence des résultats de ces analyses.

Enfin, les résultats obtenus avec les méthodes susmentionnées manquent parfois de précision. En particulier, les dispositifs connus de mesure par DSC ou par $\mu$DSC (micro-DSC) ne permettent pas de détecter des transitions peu énergétiques ou des transitions n'affectant qu'une fraction réduite de la matière analysée.

On connait aussi du document « Eliot et al., 2005, Food Hydrocolloïds, 19, 279-287. *Understanding température-sensitive caseinate emulsions : new information from diffusing wave spectroscopy* » un dispositif d'analyse spectroscopique par ondes diffusées (« *DWS, Diffusing Wave Spectroscopy* ») comprenant une source formée d'un laser He-Ne, un tube photomultiplicateur, une fibre optique formée de deux faisceaux de fibres optiques et un capteur de température. L'une des extrémités de la fibre optique est plongée dans une émulsion à analyser, les extrémités de chacun des deux faisceaux de fibres optiques sont en connexion respectivement avec la source laser He-Ne et avec le tube photomultiplicateur.

Ce dispositif ne permet pas une analyse d'une matière à analyser sans contact avec cette matière et nécessite un rinçage soigneux de la fibre optique entre deux utilisations. Ce dispositif est en outre limité dans ses applications à l'étude d'un milieu liquide susceptible de recevoir par immersion l'extrémité de la fibre optique. Il ne permet pas non plus une analyse à l'échelle macroscopique, la zone d'observation étant limitée à l'extrémité de la fibre optique. Il ne permet pas non plus une analyse d'une transition cristalline ou transition polymorphique d'une matière non purement gazeuse. Ce dispositif ne permet pas non plus d'analyser un phénomène dont la dynamique est lente, c'est-à-dire un phénomène pouvant présenter un temps de relaxation de l'ordre d'une à quelques heure(s). Il ne permet pas non plus de réaliser des variations de température avec une vitesse de variation jusqu'à 40°C/min.

Ce dispositif n'est en outre pas adapté pour pouvoir être mis en oeuvre sur des matières portées à une température extrême basse (-20°C) ou à une température extrême haute (120°C).

US 2005/0037499 A1 décrit un dispositif et une méthode afin de déterminer la température à laquelle un changement de phase d'une matière non purement gazeuse a lieu par notamment des mesures d'un rayonnement électromagnétique diffusé par une surface libre de la matière. Cependant, le suivi de la température sans contact de la surface libre de la matière n'est pas établi à la même partie de la surface libre de la matière que pour les mesures d'un rayonnement électromagnétique diffusé.

L'invention vise donc à pallier ces inconvénients.

L'invention vise à proposer un dispositif et un procédé d'analyse d'un changement d'état physique d'une matière non purement gazeuse permettant la détection d'un tel changement d'état physique affectant une partie minoritaire de la matière non purement gazeuse.

L'invention vise à proposer un dispositif et un procédé d'analyse d'un changement d'état physique d'une matière non purement gazeuse qui ne nécessitent qu'une préparation minimale d'un échantillonn de matière non purement gazeuse en vue de son analyse. En particulier, l'invention vise à proposer un tel dispositif et un tel procédé d'analyse susceptibles de pouvoir être facilement mis en oeuvre sur une matière non purement gazeuse dans son état prêt à être distribué.

L'invention vise en particulier à proposer un tel dispositif et un tel procédé d'analyse susceptibles de pouvoir être facilement mis en oeuvre sur une matière non purement gazeuse en fin de chaine d'élaboration de la matière non purement gazeuse aux fins de détecter une malfaçon, par exemple dans le cadre du contrôle qualité.

L'invention vise également à proposer un dispositif et un procédé d'analyse d'une matière non purement gazeuse pour leur utilisation dans un cadre de recherche, de développement et d'optimisation de procédés de fabrication.

En particulier l'invention vise aussi à proposer un tel dispositif et un tel procédé d'analyse susceptibles d'être appliqués pour l'analyse d'un changement d'état physique d'une matière non purement gazeuse opaque.

L'invention vise aussi à proposer un tel dispositif et un tel procédé d'analyse qui soient simples dans leur mise en oeuvre.

L'invention vise donc à proposer un tel dispositif et un tel procédé d'analyse qui ne nécessitent pas d'étape particulière de conditionnement de la matière non purement gazeuse et qui, en particulier, ne nécessitent pas une dilution de la matière non purement gazeuse.

L'invention vise aussi à proposer un tel dispositif et un tel procédé d'analyse qui ne soient pas invasifs, c'est-à-dire qui ne nécessitent pas d'établir un contact avec la matière non purement gazeuse, autre que le contact entre un support de matière à analyser et la matière à analyser elle-même.

L'invention vise à proposer un tel dispositif et un tel procédé d'analyse d'une matière non purement gazeuse pouvant être mis en oeuvre en utilisant une large gamme de température.

L'invention vise en particulier à proposer un tel dispositif et un tel procédé d'analyse pouvant être mis en oeuvre sans poser de problème de condensation de vapeur d'eau de l'air atmosphérique lors de mesures à basse température -en particulier jusqu'à -20°C - et qui sont de nature à affecter la valeur du signal mesuré.

L'invention vise en particulier à proposer un tel dispositif et un tel procédé d'analyse d'une matière non purement gazeuse -notamment d'une matière non purement gazeuse contenant de l'eau- pouvant être mis en oeuvre sans poser de problème d'effet « miroir » par convection d'air lors de mesures à haute température -en particulier jusqu'à +120°C -, et qui sont de nature à affecter la valeur du signal mesuré.

L'invention vise aussi en particulier à proposer un tel dispositif et un tel procédé d'analyse d'une matière non purement gazeuse permettant la détection d'un changement d'état physique dans un échantillon de matière non purement gazeuse de masse comprise entre 0,1 gramme et quelques grammes.

L'invention vise également à proposer un tel dispositif et un tel procédé d'analyse de la dynamique d'une matière non purement gazeuse à l'échelle microscopique affectant la structure de cette matière non purement gazeuse.

L'invention vise aussi en particulier à proposer un tel dispositif et un tel procédé pour l'analyse d'une transition cristalline -notamment d'une transition polymorphique-, c'est-à-dire d'une transition cristalline solide/solide entre une première forme cristalline d'une matière non purement gazeuse et une deuxième forme cristalline de cette matière non purement gazeuse.

Cependant, l'invention vise aussi en particulier à proposer un tel dispositif et un tel procédé d'analyse d'un changement d'état physique choisi dans le groupe formé d'un changement d'état physique solide/liquide et d'un changement d'état physique liquide/solide.

L'invention concerne un dispositif d'analyse d'un changement d'état physique d'une matière non purement gazeuse selon la revendication 1.

Selon l'invention, le dispositif d'analyse comprend un support de réception de la matière non purement gazeuse, adapté pour que cette matière portée par le support de réception présente au moins une partie de surface libre interposée entre la source et le support de réception. Un tel dispositif d'analyse permet de diriger le rayonnement électromagnétique cohérent directement sur une partie de surface libre de la matière non purement gazeuse à analyser. Un tel dispositif permet d'appliquer un rayonnement électromagnétique sur ladite partie de surface libre qui ne soit pas modifié sensiblement par rapport au rayonnement électromagnétique émis par la source. En particulier, le rayonnement électromagnétique n'est pas modifié avant son application sur la matière non purement gazeuse à analyser par un support -notamment par un support réfléchissant, diffractant, réfractant ou diffusant le rayonnement électromagnétique- interposé entre la source et ladite partie de surface libre. Le rendement d'illumination de la matière non purement gazeuse est optimisé.

La source est disposée pour diriger le rayonnement électromagnétique cohérent sur au moins une partie de surface libre de la matière portée par le support de réception et pour former un rayonnement électromagnétique diffusé par la matière dont au moins une partie est émise en direction du détecteur.

Le dispositif selon l'invention comprend un détecteur d'au moins une grandeur d'au moins une partie de rayonnement électromagnétique diffusé par la matière adapté pour pouvoir détecter ladite grandeur au cours de la variation dans le temps de la température de la matière. Un tel dispositif permet de réaliser une mesure de ladite grandeur de rayonnement électromagnétique à partir de la matière hors équilibre thermodynamique.

La source est orientée de façon que le rayonnement électromagnétique cohérent soit incident sur au moins une partie de surface libre de la matière non purement gazeuse à analyser. Un dispositif selon l'invention permet d'analyser une matière non purement gazeuse par simple interposition d'au moins une partie de surface libre de cette matière entre le support de réception et la source de rayonnement électromagnétique cohérent. Un tel dispositif est donc particulièrement simple d'utilisation.

Avantageusement et selon l'invention, le support de réception est un porte échantillon adapté pour recevoir la matière non purement gazeuse à analyser. Un tel support de réception est adapté pour recevoir la matière à analyser et la source est dirigée pour former un rayonnement électromagnétique pour permettre une illumination d'au moins une partie de surface libre de la matière à analyser par le rayonnement électromagnétique cohérent produit par la source.

Avantageusement et selon l'invention, le dispositif est adapté pour permettre une pluralité de mesures de lumière diffusée par la matière non purement gazeuse au cours d'une variation de la température de la matière non purement gazeuse.

Dans tout le texte, les expressions « diffusion d'un rayonnement électromagnétique », « rayonnement électromagnétique diffusé » et « rayonnement électromagnétique rétrodiffusé » sont relatives au phénomène physique bien connu par lequel un rayonnement électromagnétique interagissant avec une matière est dévié de sa propagation rectiligne dans un volume de la matière, en particulier par un constituant (particule, goutte, micelle, polymère, agrégat) présent dans ce volume de cette matière. L'expression « diffusion multiple » représente la réitération du phénomène de diffusion dans des systèmes concentrés et/ou optiquement contrastés. En particulier, cette déviation d'un rayonnement électromagnétique dans ce volume de la matière est distincte d'une réflexion spéculaire, d'une réflexion diffuse et d'une réfraction de lumière se produisant à l'interface de deux milieux distincts.

L'invention vise un dispositif et un procédé d'analyse d'un changement d'état physique d'une matière non purement gazeuse par analyse d'un rayonnement électromagnétique diffusé -notamment rétrodiffusé- par cette matière, notamment par diffusions multiples dans au moins une partie du volume de la matière. L'invention ne vise donc pas un dispositif et un procédé d'analyse d'un changement d'état physique d'une telle matière par analyse d'un rayonnement électromagnétique essentiellement réfléchi par une interface air/matière.

L'inventeur a constaté qu'il est possible de détecter un changement de structure intervenant dans une matière à l'échelle nanométrique et de caractériser un changement d'état physique d'une matière non purement gazeuse, en particulier une transition cristalline d'une matière non purement gazeuse, en analysant la vitesse de variation -notamment la vitesse de fluctuation- d'une grandeur du rayonnement électromagnétique diffusé par la matière soumise à un rayonnement électromagnétique incident et à une variation temporelle de température. Il a observé, de façon totalement surprenante, qu'il est possible de réaliser des mesures sans contact d'un rayonnement électromagnétique diffusé par au moins une partie de surface libre d'une matière non purement gazeuse soumise sans contact à un rayonnement électromagnétique incident et à une variation temporelle de température et de détecter un changement d'état physique, en particulier une transition de phase ou une transition cristalline d'une matière non purement gazeuse se produisant dans cette matière non purement gazeuse lorsque cette matière est soumise à cette variation temporelle de température. Avantageusement selon certains modes de réalisation, la variation temporelle de la température de la matière non purement gazeuse peut être une rampe temporelle de variation de température prédéterminée.

Le dispositif selon l'invention permet une analyse d'un changement d'état physique d'une matière non purement gazeuse susceptible d'intervenir sur une fraction seulement de la matière. Le dispositif selon l'invention permet une analyse d'un changement -notamment d'une transitiond'état physique n'affectant qu'une partie minoritaire de la matière non purement gazeuse.

Le dispositif selon l'invention permet une analyse non invasive d'un changement d'état physique et qui peut être mise en oeuvre sur simple présentation d'un échantillon de matière dans le dispositif selon l'invention. Dans un dispositif selon l'invention, la source de rayonnement électromagnétique est placée à distance de la matière à analyser et sans contact avec la matière à analyser. Ainsi, la source de rayonnement électromagnétique ne nécessite pas d'opération de nettoyage de la source lors d'analyses successives de matières à analyser de nature différente.

La source de rayonnement électromagnétique incident est une source d'un rayonnement électromagnétique choisie pour former une image d'interférence.

La source de rayonnement électromagnétique incident est une source d'un rayonnement électromagnétique -notamment une source d'un rayonnement lumineux- cohérent. Avantageusement, la source de rayonnement électromagnétique incident est une source d'un rayonnement électromagnétique spatialement cohérent. Il peut s'agir d'une source de rayonnement électromagnétique formant un faisceau de rayonnement électromagnétique sensiblement parallèle, faiblement convergent ou faiblement divergent.

Avantageusement, le rayonnement électromagnétique incident peut être sous la forme d'un faisceau de rayonnement électromagnétique incident faiblement divergent, c'est-à-dire présentant une divergence de valeur d'angle comprise entre 1° et 3°. La surface de la tache formée sur la matière par le rayonnement électromagnétique incident présente des dimensions millimétriques. La surface de la tache formée sur la matière par le rayonnement électromagnétique incident peut présenter une aire de l'ordre d'un $cm^2$. Ainsi, avantageusement et selon l'invention, ladite au moins une partie de surface libre de la matière non purement gazeuse de réception du rayonnement électromagnétique cohérent est de dimension millimétrique. De telles dimensions de la tache formée en surface de la matière permettent d'effectuer des mesures représentatives de la totalité de la matière.

La source de rayonnement électromagnétique incident est une source d'un rayonnement électromagnétique temporellement cohérent. Le rayonnement électromagnétique temporellement cohérent peut présenter une dispersion spectrale autour d'une valeur moyenne de longueur d'onde comprise entre 1 nm et 50 nm.

Avantageusement et selon l'invention, la source de rayonnement électromagnétique incident est une source de rayonnement laser. Avantageusement, la source de rayonnement électromagnétique incident est une source de rayonnement laser -notamment une diode laser- d'émission d'ondes électromagnétiques à une longueur d'onde comprise entre 400 nm et 800 nm, notamment comprise entre 600 nm et 700 nm, en particulier de l'ordre de 650 nm. Avantageusement, la source de rayonnement laser de longueur d'onde de l'ordre de 650 nm.

Avantageusement, la source de rayonnement électromagnétique du dispositif selon l'invention est une source de rayonnement laser de longueur d'onde et de puissance de valeurs choisies pour limiter le réchauffement de la matière par le rayonnement électromagnétique et préserver la matière d'une modification et une dénaturation de sa structure chimique.

Avantageusement, le dispositif selon l'invention présente une lentille convexe interposée sur le faisceau de rayonnement électromagnétique incident entre la source et la matière non purement gazeuse.

Dans un dispositif selon l'invention, le rayonnement électromagnétique cohérent dirigé sur ladite au moins une partie de surface libre de la matière est de parcours libre entre la source et la matière non purement gazeuse.

Avantageusement et selon l'invention, le détecteur peut être choisi dans le groupe formé d'un tube photomultiplicateur, d'une photodiode et d'un capteur matriciel.

Avantageusement, selon un mode de réalisation particulier de l'invention, le détecteur est un tube photomultiplicateur. Un tel tube photomultiplicateur permet de mesurer l'évolution temporelle d'une grandeur telle que l'intensité du rayonnement électromagnétique -notamment l'intensité lumineuse- diffusé par la matière non purement gazeuse.

Avantageusement, selon un autre mode de réalisation préférentiel de l'invention, le capteur matriciel est choisi dans le groupe formé d'un capteur matriciel CCD (« *Coupled-Charged Device* ») et d'un capteur matriciel CMOS (« *Complementary Metal-Oxide Semiconductor* »). Un dispositif selon l'invention comprenant un capteur matriciel (CCD ou CMOS) permet de mesurer l'évolution temporelle d'une grandeur telle que l'image du rayonnement électromagnétique -notamment de la lumière- diffusé par la matière non purement gazeuse à analyser.

Avantageusement, le détecteur est disposé à distance de la matière non purement gazeuse et sans contact avec cette dernière de façon à recevoir ladite au moins une partie de rayonnement électromagnétique diffusé.

Dans un dispositif selon l'invention, le détecteur est placé à distance de la partie de surface libre de matière à analyser et sans contact avec ladite partie de surface libre de matière à analyser. Ainsi, d'une part, le détecteur ne nécessite pas d'opération de nettoyage du détecteur lors d'analyses successives de matières à analyser de nature différente. D'autre part, le dispositif selon l'invention permet une analyse non invasive d'un changement d'état physique et qui peut être mise en oeuvre simplement par présentation d'un échantillon de matière dans le dispositif selon l'invention. En outre, la position de la source à distance de la matière à analyser et sans contact avec cette matière permet une illumination d'au moins une partie de matière et une analyse de cette partie de matière qui est représentative de la totalité de la matière. Le dispositif selon l'invention permet donc une analyse d'une matière non purement gazeuse sans nécessiter un prélèvement d'un échantillon et son conditionnement dans une enceinte susceptibles de modifier les propriétés de la matière à analyser et de fausser les résultats de cette analyse.

Avantageusement, dans un dispositif selon l'invention, la source de rayonnement électromagnétique incident, la matière à analyser et le détecteur sont positionnés pour minimiser l'intensité du rayonnement électromagnétique réfléchi -par réflexion spéculaire et/ou réflexion diffuse- par la matière et collecté par le détecteur.

Avantageusement, dans une première variante d'un dispositif selon l'invention, le détecteur est positionné dans le demi-espace défini par le plan de présentation de la matière et comprenant la source de rayonnement électromagnétique, de façon à collecter un rayonnement électromagnétique rétrodiffusé par la matière qui est sensiblement dépourvu de rayonnement électromagnétique réfléchi par la matière. Cependant, rien n'exclut, dans une autre variante d'un dispositif selon l'invention, que le détecteur soit positionné dans le demi-espace défini par le plan de présentation de la matière et ne comprenant pas la source de rayonnement électromagnétique incident et de façon à recevoir un rayonnement électromagnétique transmis par la matière à analyser.

Avantageusement, dans un mode de réalisation préférentiel :

- le dispositif comprend un porte échantillon adapté pour recevoir une coupelle de mesure présentant un fond sensiblement plan de réception de la matière à analyser, le fond plan étant disposé horizontalement dans le dispositif selon l'invention ;
- le détecteur est positionné à la verticale de la partie de matière à analyser recevant le rayonnement électromagnétique incident ;
- la source de rayonnement électromagnétique est positionnée de sorte que le rayonnement électromagnétique rétrodiffusé par la matière et collecté par le détecteur forme avec le rayonnement électromagnétique incident un angle de valeur comprise entre 10° et 20°, en particulier de l'ordre de 13°.

Ainsi, dans ce mode de réalisation préférentiel, le dispositif selon l'invention permet la collecte et l'analyse d'un rayonnement électromagnétique rétrodiffusé par la matière à analyser, le rayonnement électromagnétique rétrodiffusé étant sensiblement exempt de rayonnement électromagnétique réfléchi par la matière.

Avantageusement, le support de présentation d'au moins une partie de surface libre de la matière non purement gazeuse comprend une platine de contrôle de la température de la matière. Avantageusement et selon l'invention, le dispositif de contrôle de la température de la matière est un dispositif d'ajustement de la température par conduction.

Avantageusement et selon l'invention, le dispositif de contrôle de la température de la matière est un dispositif à effet Peltier (à effet thermoélectrique). Avantageusement, le dispositif de contrôle de la température de la matière est adapté pour pouvoir contrôler la température de la matière non purement gazeuse et faire varier la température de la matière non purement gazeuse selon une variation temporelle de température. Le dispositif de contrôle de la température permet un contrôle de la température de la matière entre -20°C et +120°C.

Le dispositif selon l'invention est aussi conçu pour permettre une telle variation de la température de la matière non purement gazeuse, tout en limitant la variation de la température de l'air atmosphérique présent entre la matière non purement gazeuse et la source de rayonnement magnétique cohérent et entre la matière non purement gazeuse et le détecteur. Le dispositif et le procédé d'analyse d'une matière non purement gazeuse est configuré pour éviter la condensation de vapeur sur le détecteur lors de mesures à basse température -en particulier jusqu'à -20°C- et un effet « miroir » par convection d'air lors de mesures à haute température -en particulier jusqu'à +120°C -. Le dispositif selon l'invention présente une pluralité d'enveloppes entourant et confinant la matière non purement gazeuse.

En outre, le dispositif selon l'invention présente une optique simplifiée, voire sensiblement inexistante. Une telle optique simplifiée est particulièrement adaptée lors de la mise en oeuvre d'une variation temporelle de température et évite la formation -notamment à basse température- de condensats perturbateurs de la mesure.

Avantageusement et selon l'invention, la variation temporelle de température de la matière non purement gazeuse répond à une rampe temporelle de variation linéaire de température de température dont la valeur absolue de la pente peut être comprise entre 0,1°C/min et 40°C/min. Avantageusement, la rampe temporelle de variation de température est une rampe -notamment une rampe linéaire- de température croissante. Avantageusement, la rampe temporelle de variation de température est une rampe -notamment une rampe linéaire- de température décroissante. Il est aussi possible que la rampe temporelle de variation de température soit une rampe de température plus complexe, présentant au moins une rampe -notamment une rampe linéaire- de température croissante et/ou au moins une rampe -notamment une rampe linéaire- de température décroissante et/ou au moins une rampe -notamment une rampe linéaire- de température constante.

Rien n'empêche également de réaliser des cycles successifs de rampes temporelles de variation de température. De tels cycles successifs permettent d'étudier, par exemple, la stabilité de la matière non purement gazeuse à analyser au cours des cycles successifs.

Avantageusement, le contrôle de la température est effectué avec une précision de l'ordre de 0,1°C.

Avantageusement, le dispositif d'analyse selon l'invention, comprend des moyens électroniques de programmation de la variation temporelle de température. Ainsi, le dispositif selon l'invention permet de définir un programme de variation temporelle de température prédéterminée dédiée à l'analyse d'une matière purement non gazeuse prédéterminée.

Selon l'invention, le dispositif d'analyse comprend des moyens de mesure sans contact de la température -notamment de la température en surface- de ladite partie de surface libre de la matière non purement gazeuse. Les moyens de mesure sans contact de la température de la matière sont adaptés pour permettre une mesure de la température de la matière à l'endroit de l'émission du rayonnement électromagnétique diffusé par la matière et de sa mesure et sans interférence avec la mesure du rayonnement électromagnétique diffusé.

Le dispositif selon l'invention est aussi conçu pour :

- faire varier la température de la matière non purement gazeuse et pour permettre une mesure par le détecteur d'au moins une grandeur d'au moins une partie de rayonnement électromagnétique diffusé par au moins une partie de surface libre de la matière non purement gazeuse au cours de la variation de température, ladite partie de surface libre de la matière non purement gazeuse étant hors équilibre thermodynamique, et ;
- pour permettre une mesure de la température de ladite partie de matière non purement gazeuse au cours de la variation de température.

Avantageusement et selon l'invention, les moyens de mesure sans contact de la température comprennent un capteur de rayonnement infrarouge. Les moyens de mesure sans contact de la température permettent de mesurer la température d'au moins une partie de surface libre de matière non purement gazeuse et sans contact avec ladite au moins une partie de surface libre. Le dispositif selon l'invention permet une mesure directe de la température de ladite partie de matière non purement gazeuse lors de la variation temporelle de la température de la matière non purement gazeuse. Ainsi, la mesure du rayonnement électromagnétique diffusé caractérise la partie de surface libre de matière non purement gazeuse dont la température est précisément mesurée au cours de la variation de température.

Les moyens de mesure sans contact de la température permettent une mesure de la température de la partie de surface libre de la matière non purement gazeuse recevant le rayonnement électromagnétique cohérent et émettant le rayonnement électromagnétique diffusé au cours de la variation de température de la matière et permettent de déterminer une température de changement d'état physique de la matière non purement gazeuse en s'affranchissant de l'inertie thermique de la matière non purement gazeuse.

Avantageusement et selon l'invention, la matière non purement gazeuse est une matière hétérogène. La matière non purement gazeuse peut-être une matière essentiellement solide. La matière non purement gazeuse peut-être une matière complexe. La matière non purement gazeuse peut présenter au moins une phase solide et au moins une phase liquide. La matière non purement gazeuse peut présenter au moins deux phases solides. Avantageusement et selon l'invention, la matière non purement gazeuse est une matière comprenant au moins un corps gras. Il peut s'agir par exemple d'une cire, de beurre, de chocolat, de rouge à lèvre, de fond de teint, de mascara, de baume, d'une huile d'origine végétale et d'une huile d'origine animale. Avantageusement et selon l'invention, la matière non purement gazeuse est une denrée alimentaire, par exemple du caramel, du miel, du fromage. Avantageusement et selon l'invention, la matière non purement gazeuse est une matière grasse.

L'invention s'étend également à un procédé d'analyse d'un changement d'état physique d'une matière non purement gazeuse, dans lequel on utilise un dispositif selon l'invention.

L'invention s'étend également à un procédé d'analyse d'un changement d'état physique d'une matière non purement gazeuse selon la revendication 6.

Dans un procédé selon l'invention on réalise la pluralité de mesures au cours de la variation de température de la matière non purement gazeuse. On réalise la pluralité de mesures sur la matière non purement gazeuse hors équilibre thermodynamique et une analyse du réarrangement microscopique affectant la structure d'une matière non purement gazeuse par diffusion dynamique de la lumière.

L'invention s'étend également à un procédé d'analyse d'un changement d'état physique d'une matière non purement gazeuse susceptible de subir un changement d'état physique sous l'effet d'une variation temporelle de température.

Dans un procédé selon l'invention, on dirige un rayonnement électromagnétique -notamment un rayonnement laser- sur une zone de surface de la matière à analyser, ladite matière étant soumise à une variation temporelle de sa température. Simultanément à cette variation temporelle de température, on réalise un enregistrement continu ou discontinu des valeurs prises par une grandeur caractéristique du rayonnement électromagnétique diffusé par la matière à analyser. Il peut s'agir d'une pluralité de valeurs d'intensité de rayonnement électromagnétique diffusé par la matière à analyser mesurée au moyen d'un détecteur du type tube photomultiplicateur ou tube de photodiode.

Avantageusement et selon l'invention, au moins une grandeur du rayonnement électromagnétique diffusé est une image d'au moins une partie du rayonnement électromagnétique diffusé. Avantageusement, chaque image de la pluralité d'images est formée sur un capteur matriciel à une valeur de temps de la rampe temporelle de variation de température et à une valeur de température de la matière à analyser déterminée par la rampe temporelle de variation de température.

Avantageusement, chaque image de la pluralité d'images est une image d'interférence (aussi nommée image de « *speckle* ») représentative des interférences résultant des différents rayonnements électromagnétiques diffusés par la matière non purement gazeuse. Les images de diffusion d'ondes électromagnétiques peuvent être enregistrées au moyen d'un capteur matriciel tel qu'un capteur CCD (« *Charge-Coupled Device* ») ou un capteur CMOS (« *Complementary Metal Oxyde Semi-conductor* » ou par tout autre moyen. Dans un procédé selon l'invention, le capteur peut être positionné en transmission, c'est-à-dire positionné à l'opposé de la source de rayonnement magnétique cohérent par rapport à la matière non purement gazeuse à analyser.

Avantageusement dans un mode de réalisation selon l'invention, au moins une grandeur du rayonnement électromagnétique diffusé est une image d'au moins une partie d'un rayonnement électromagnétique rétrodiffusé. Le capteur peut aussi être positionné pour capter un rayonnement électromagnétique rétrodiffusé par la matière à analyser, c'est-à-dire positionné dans le même demi espace que la source de rayonnement électromagnétique incident, ledit demi espace étant défini par le plan comprenant le support de matière à analyser. Le capteur est positionné de façon que les axes du rayonnement électromagnétique incident et du rayonnement électromagnétique diffusé par la matière à analyser et reçu par le capteur soient sécants et de façon que le rayonnement électromagnétique incident et le rayonnement électromagnétique diffusé par la matière à analyser et reçu par le capteur forment un angle aigu, en particulier un angle de valeur comprise entre 10° et 20°. Cette configuration permet notamment mais non exclusivement d'analyser une matière non purement gazeuse opaque par nature, présentant une dynamique lente, une viscosité élevée ou un caractère élastique, voire solide.

Avantageusement et selon l'invention ;

  ∘ on compare chaque image $I_n$ de la pluralité d'images mesurées avec une image $I_a$ antérieure de la pluralité d'images mesurées ;

∘ on calcule une valeur, dite distance inter-image $d_2$, représentative des différences que présente l'image $I_n$ en comparaison avec l'image $I_a$, puis ;

o on analyse l'évolution de ladite distance inter-image $d_2$ sur chaque intervalle de temps d'une pluralité d'intervalles de temps au cours desquels ont fait varier la température de la matière, et ;

o on déduit la valeur représentative de la vitesse de variation -notamment de la vitesse de fluctuation- de la distance inter-image $d_2$ sur chaque intervalle de temps, ladite valeur étant représentative du changement d'état physique de la matière.

Avantageusement et selon l'invention :

o on calcule, pour chaque image $I_n$ de chaque intervalle de temps d'une pluralité d'intervalles de temps, une valeur, dite distance inter-image $d_2$, représentative de la variation détectée entre ladite image $I_n$ de rang n dans la pluralité d'images mesurées et une image $I_{n-m}$ de rang n-m dans la pluralité d'images mesurées, n et m étant deux entiers naturels tels que n > m, puis ;

o on trace l'évolution de la distance inter-image $d_2$ sur chaque intervalle de temps ;

∘ on déduit ladite valeur représentative de la vitesse de variation -notamment de la vitesse de fluctuation- de la distance inter-image $d_2$ sur chaque intervalle de temps, ladite valeur étant représentative du changement d'état physique de la matière.

Avantageusement, selon une variante d'un procédé selon l'invention, la distance inter-image $d_2$ est représentative de la variation détectée entre deux images $I_n$ et $I_{n-1}$ successives de la pluralité d'images mesurées sur l'intervalle de temps considéré de la pluralité d'intervalles.

Avantageusement, selon une autre variante d'un procédé selon l'invention, la distance inter-image $d_2$ est représentative de la variation détectée entre une image $I_n$ de la pluralité d'images mesurées et la première image de la pluralité d'images mesurées sur l'intervalle de temps considéré de la pluralité d'intervalle. On compare à elle-même la première image mesurée sur l'intervalle de temps considéré et on attribue à la première image une valeur de distance inter-image $d_2$ nulle.

Selon l'invention, on mesure la température en surface de la matière non purement gazeuse au cours de la variation temporelle de la température.

Avantageusement et selon l'invention, on fait varier la température de la matière selon une rampe temporelle de température choisie dans le groupe formé des rampes de température croissante, des rampes de température décroissante, des rampes de température présentant alternativement des phases de température croissante et des phases de température décroissante et des rampes de température présentant au moins une phase de température croissante, et/ou au moins une phase de température décroissante et, la cas échéant, au moins un plateau de stabilisation à température constante.

Avantageusement et selon l'invention, ladite au moins une partie du rayonnement électromagnétique diffusé par la matière non purement gazeuse est un rayonnement électromagnétique rétrodiffusé par la matière non purement gazeuse.

Avantageusement, le procédé selon l'invention est mis en oeuvre sur une chaîne d'élaboration de ladite matière non purement gazeuse. Avantageusement, dans un procédé selon l'invention, on analyse la matière non purement gazeuse sans échantillonnage de la matière susceptible de fausser l'analyse. Avantageusement, dans un procédé selon l'invention, on prélève un fragment de matière non purement gazeuse dont l'apparence est identique à la matière non purement gazeuse produite sur la chaîne d'élaboration et on analyse le fragment de matière non purement gazeuse sans modification préalable de l'apparence dudit fragment.

Avantageusement et selon l'invention, la matière non purement gazeuse est une matière solide. Avantageusement, la matière non purement gazeuse comprend un corps gras. Avantageusement et selon l'invention, la matière non purement gazeuse est un corps gras. Avantageusement et selon l'invention, la matière non purement gazeuse est du chocolat.

L'invention s'étend également à l'utilisation d'un dispositif et/ou un procédé selon l'invention pour l'analyse d'un changement d'état physique d'une matière non purement gazeuse.

L'invention s'étend par exemple à l'utilisation d'un dispositif et/ou un procédé selon l'invention pour l'analyse d'un changement d'état physique d'une matière non purement gazeuse choisie dans le groupe formé d'une cire, de beurre, de chocolat, de rouge à lèvre, de fond de teint, de mascara, de baume, d'une huile d'origine végétale et d'une huile d'origine animale.

L'invention s'étend également et en particulier à l'utilisation d'un dispositif et/ou un procédé selon l'invention pour analyser un changement d'état physique de chocolat permettant d'évaluer un risque de survenue d'un blanchiment gras de surface de chocolat. L'invention s'étend également à l'utilisation d'un dispositif selon l'invention pour évaluer un risque de survenue d'un blanchiment gras de chocolat à toute étape d'élaboration de ce chocolat.

L'invention concerne donc un un dispositif selon la revendication 1 et un procédé selon la revendication 6 d'analyse d'un changement d'état physique d'une matière non purement gazeuse, ainsi que l'utilisation du dispositif selon la revendication 1 pour prédire la survenue d'un blanchiment gras de chocolat selon la revendication 13.

D'autres buts, caractéristiques et avantages de l'invention apparaîtront à la lecture de la description suivante donnée à titre uniquement non limitatif et qui se réfère aux figures annexées, dans lesquelles :

- la figure 1 est une représentation en perspective d'un dispositif conforme à l'invention débarrassé de son capot de protection ;
- la figure 2 est une représentation en perspective d'un dispositif conforme à l'invention avec son capot de protection ;
- la figure 3 est une représentation graphique d'une analyse d'un changement d'état physique de chocolat correctement tempéré (♦) ou incorrectement tempéré (O) en fonction de la température, réalisée avec un dispositif selon l'invention, et ;
- la figure 4 est une représentation graphique d'une analyse d'un changement d'état physique de chocolat correctement tempéré (♦) ou incorrectement tempéré (O) en fonction de la température, réalisée avec un dispositif selon l'invention.

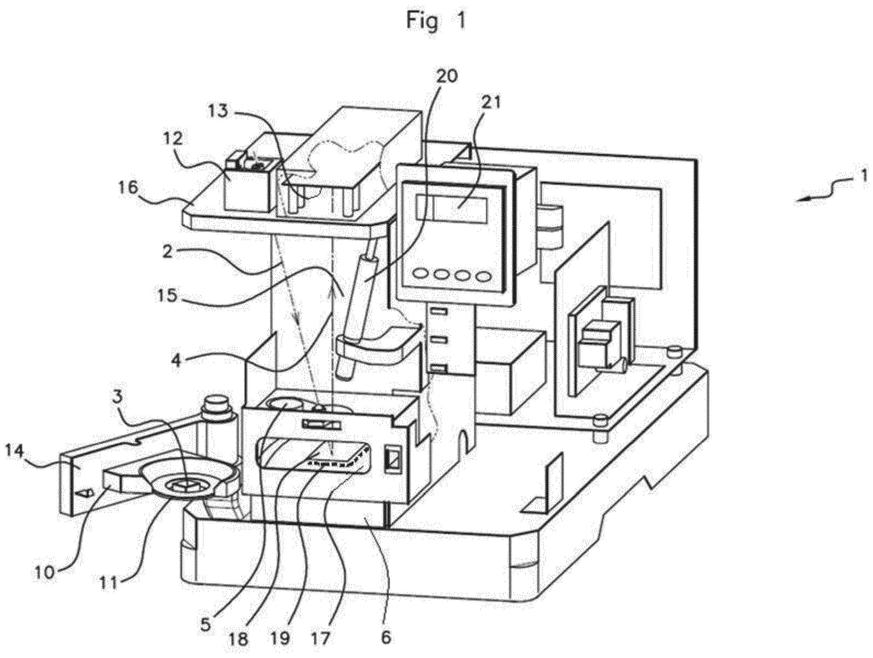

Un dispositif 1 selon l'invention représenté en figure 1 comprend un porte-échantillon 10 adapté pour recevoir une coupelle 11 de mesure et placer la coupelle 11 de mesure et une matière 3 à analyser contenue dans la coupelle 11 en position de mesure dans le dispositif 1. Dans cette position de mesure, la matière 3 à analyser est placée pour pouvoir recevoir un rayonnement électromagnétique incident représenté par son axe 2 de propagation et délivré par une source de rayonnement électromagnétique formée d'une diode 12 laser. Dans la position de mesure, la matière 3 à analyser est aussi positionnée pour qu'au moins une partie du rayonnement électromagnétique diffusé par la matière à analyser selon un axe, dit axe 4 de rétrodiffusion, sous l'effet du rayonnement électromagnétique incident soit reçue par un détecteur 13 de rayonnement électromagnétique diffusé. Le détecteur 13 peut comprendre un capteur matriciel, par exemple une caméra CCD ou une caméra CMOS, équipé d'une carte d'acquisition vidéo. Le capteur matriciel peut-être de toute forme et de toutes dimensions. Le détecteur 13 est adapté pour permettre l'acquisition d'images avec une fréquence élevée, par exemple comprise entre 40 images par seconde et 400 images par seconde.

Le porte-échantillon 10 est monté solidaire d'un battant 14 adapté pour, dans une position ouverte du battant 14, permettre le chargement de la matière à analyser dans la coupelle 11 de mesure, et, dans une position fermée du battant 14, mettre la matière à analyser en position d'analyse dans une enceinte 17 de mesure close.

Le dispositif 1 selon l'invention comprend un obturateur 5 adapté pour pouvoir obturer un orifice d'entrée du rayonnement électromagnétique incident dans une enceinte 17 de mesure lorsque le battant 14 est en position ouverte et libérer l'orifice d'entrée du rayonnement électromagnétique incident dans l'enceinte 17 de mesure lorsque le battant 14 est en position fermée.

Le dispositif 1 selon l'invention comprend une platine 16 support de la diode 12 laser et du détecteur 13. La platine 16 présente un orifice de passage d'un rayonnement électromagnétique incident et un orifice de passage d'une partie de rayonnement électromagnétique diffusé par la matière à analyser. La diode 12 laser est munie d'une lentille interposée sur le trajet séparant la lentille et la matière à analyser et adaptée pour produire un faisceau incident faiblement divergent. Dans un dispositif 1 selon l'invention, aucun élément d'optique n'est nécessaire pour la collecte de la partie de rayonnement électromagnétique diffusé par la matière à analyser. La diode 12 laser peut être, par exemple, une diode laser émettant à la longueur d'onde de 650 nm. Dans ces conditions, le dispositif selon l'invention et la diode 12 laser ne présente pas de danger pour l'utilisateur, notamment pour les yeux de l'utilisateur.

Le dispositif 1 selon l'invention comprend une enceinte 17 de mesure adaptée pour recevoir la coupelle 11 de mesure lorsque le battant 14 est en position de fermeture et pour limiter les mouvements d'air autour de la matière à analyser au cours des rampes temporelles de variation de température. L'enceinte 17 est formée d'un polymère, par exemple de poly(oxyméthylène). L'enceinte 17 présente un orifice ménagé pour former un passage du rayonnement électromagnétique cohérent incident et d'au moins une partie du rayonnement électromagnétique diffusé, en particulier rétrodiffusé.

Le dispositif 1 selon l'invention comprend un dispositif de contrôle et d'ajustement de la température de la matière à analyser selon une rampe de variation de température prédéterminée. Le dispositif de contrôle et d'ajustement de la température comprend un organe 19 de chauffage/refroidissement de la matière à analyser surmonté d'une platine 18 en aluminium sur laquelle la coupelle 11 de mesure vient en contact lorsque le battant 14 est en position de fermeture de l'enceinte 17 de mesure. L'organe 19 de chauffage/refroidissement de la matière à analyser peut être un organe 19 à effet thermoélectrique -du type Peltier-. La platine 18 et l'organe 19 thermoélectrique sont dimensionnés pour que la platine 18 recouvre l'organe 19 thermoélectrique. Ainsi, dans cette configuration, l'énergie fournie par l'organe 19 thermoélectrique est transmise efficacement à la platine, à la coupelle 11 de mesure (lorsque le battant 14 est en position de fermeture) et à la matière 3 non purement gazeuse. L'organe 19 thermoélectrique s'étendant sous-jacent à la platine 18 est en outre entouré d'un matériau isolant adapté pour s'opposer à une dissipation latérale de l'énergie fournie par l'organe 19 thermoélectrique. Le dispositif 1 selon l'invention présente en outre, sous-jacent à l'organe 19 thermoélectrique, un bloc 6 de dissipation de l'énergie fournie par la face inférieure de l'organe 19 thermoélectrique. Le bloc 6 de dissipation peut-être un bloc en aluminium. Le dispositif 1 selon l'invention forme donc une enceinte 17 de mesure dans laquelle l'énergie fournie par l'organe thermoélectrique est concentrée sur la coupelle 11 de mesure via la platine 18. L'enceinte 17 de mesure forment donc une première enceinte isolante s'opposant à la dissipation de l'énergie fournie par l'organe 19 thermoélectrique à l'extérieur de l'enceinte 17 de mesure. Le dispositif 1 selon l'invention présente aussi un espace 15 de cheminement du rayonnement 2 électromagnétique cohérent émis par la source 12 et du rayonnement 4 électromagnétique diffusé par la matière 3 non purement gazeuse, ledit espace 15 de cheminement s'étendant entre l'enceinte 17 de mesure et la platine 16 et formant une deuxième enceinte isolante. Le dispositif 1 selon l'invention présente enfin un capot 7 externe (non représenté en figure 1) et formant une troisième enceinte isolante. Le dispositif 1 selon l'invention est muni de trois enceintes isolantes permettant de faire varier la température de la matière non purement gazeuse en confinant cette variation de température à l'enceinte 17 de mesure et en évitant de faire varier sensiblement la température dans l'espace 15 de cheminement.

Dans la configuration d'un dispositif 1 selon l'invention représenté en figure 1, la régulation de la température de la matière placée dans la coupelle 11 de mesure est obtenue par chauffage/refroidissement par le fond de la coupelle 11 de mesure et non par les parois latérales de ladite coupelle 11 de mesure. Le dispositif selon l'invention permet la formation d'un gradient de température dirigé à partir du fond de la coupelle en direction de la partie supérieure de la matière à analyser et non d'un gradient de température dirigé à partir de la paroi latérale en direction du centre de la coupelle 11 de mesure. Ainsi, la couche de surface de matière à analyser recevant le rayonnement électromagnétique incident et émettant le rayonnement électromagnétique diffusé par la matière à analyser présente une température sensiblement homogène sur toute sa surface et permet des mesures du rayonnement électromagnétique diffusé en minimisant les effets d'un gradient radial de température dans la coupelle 11 de mesure à l'origine d'un gradient optique susceptible de fausser la mesure.

Le dispositif d'ajustement de la température de la matière à analyser comprend en outre des moyens de programmation de l'organe 19 de chauffage/refroidissement et de contrôle en retour de la température de la platine 18 adaptés pour permettre la programmation de rampes temporelles de variation de température et une régulation efficace de la température de la platine 18. Le dispositif d'ajustement de la température de la matière à analyser est choisi pour pouvoir former des rampes temporelles de variation de température croissante, ou des rampes temporelles de variation de température décroissante, ou tout autre rampe temporelle de variation de température complexe, notamment des rampes temporelles de variation de température comprenant des phases de température croissante, des phases de température décroissante et/ou des fins de température constante. Rien n'empêche également de réaliser des cycles successifs de rampes temporelles de variation de température. De tels cycles successifs permettent d'étudier, par exemple, la stabilité de la matière non purement gazeuse à analyser.

Le dispositif 1 selon l'invention comprend un capteur 20 de température adapté pour pouvoir mesurer la température d'une couche de surface extérieure de la matière à analyser au cours de la rampe temporelle de variation de température. Le capteur 20 de température peut-être de tout type. Il peut s'agir d'un capteur 20 de température sans contact, adapté pour pouvoir mesurer la température de la matière à analyser sans pour autant perturber la mesure du rayonnement électromagnétique diffusé. En particulier, le capteur 20 de température peut-être un capteur à infrarouge.

Le dispositif 1 selon l'invention est équipé de pieds antivibration conçus pour minimiser l'influence de vibrations externes au dispositif 1 et améliorer la détermination de la distance inter-image $d_2$. Le dispositif 1 selon l'invention présente aussi en façade un organe 21 d'affichage de la température de la matière en cours d'analyse.

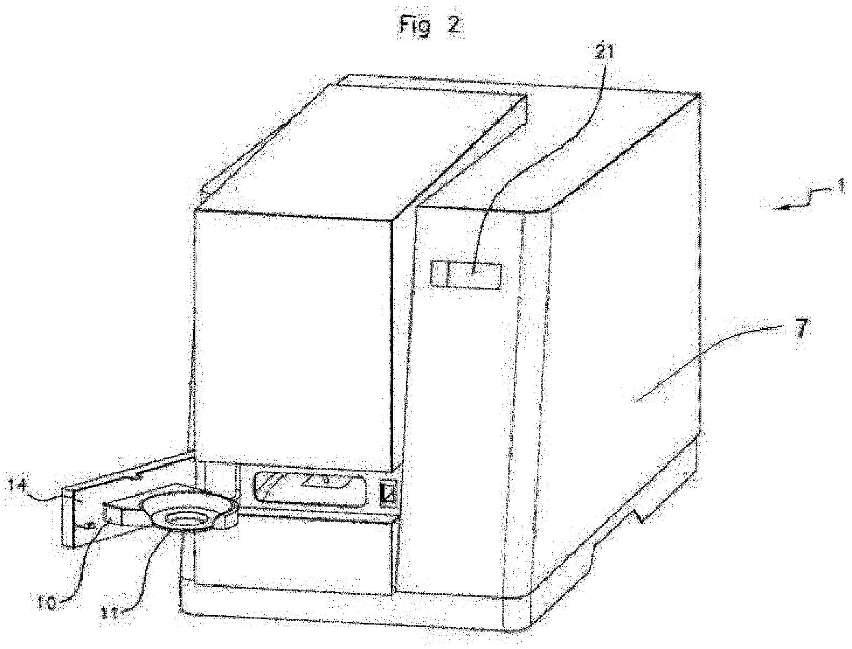

Une variante d'un dispositif 1 selon l'invention est représentée en figure 2 avec son capot 7 de protection, le battant 14 étant en position ouverte. Ne sont pas représentées en figure 2, les câbles d'alimentation électrique et de connexion du dispositif selon l'invention avec un dispositif électronique de transfert, de visualisation, et de traitement des données.

Dans un procédé selon l'invention, on soumet la matière à analyser à une rampe temporelle de variation de température et on réalise au cours du temps et au cours de cette rampe temporelle de variation de température l'acquisition de mesures successives d'une grandeur représentative du rayonnement électromagnétique rétrodiffusé par la matière analysée. Il peut s'agir d'une mesure de l'intensité du rayonnement électromagnétique rétrodiffusé par la matière analysée. Il peut cependant aussi s'agir d'une représentation en deux dimensions de la répartition topologique du rayonnement électromagnétique rétrodiffusé par la matière analysée sur un capteur matriciel.

Dans le cas d'une analyse d'images matricielles, on calcule une valeur, dite distance inter-image $d_2$ (ou distance vectorielle quadratique) représentative d'une variation (ou « décorrélation ») entre deux images successives de la pluralité d'images, selon la formule (I) suivante :

$$d_2 = \sqrt{\sum [I_2(x,y) - I_1(x,y)]^2} \qquad \text{(I)}$$

(I) dans laquelle ;

- $I_2(x,y)$ représente l'intensité lumineuse d'une image $I_2$ mesurée par un pixel de coordonnées (x,y) du capteur matriciel, et ;
- $I_1(x,y)$ représente l'intensité lumineuse d'une image $I_1$ mesurée par un pixel de coordonnées (x,y) du capteur matriciel.

Il est aussi possible de calculer une distance inter-image $d_2$ représentative de la variation entre une image mesurée de la pluralité d'images mesurées sur un intervalle de temps de la pluralité d'intervalles de temps et la première image mesurée sur cet intervalle de temps de la pluralité d'intervalles de temps.

La distance inter-image $d_2$ est par définition un nombre non négatif qui permet de quantifier la variation intervenant entre deux images formées à différents temps ou à différentes températures de la rampe de températures. Si les deux images sont identiques, la distance inter-image $d_2$ de ces deux images est nulle. Si les deux images diffèrent, la distance inter-image $d_2$ de ces deux images est non nulle et augmente lorsque la différence entre les deux images augmente.

Dans un procédé selon l'invention, on détermine des intervalles de temps, chaque intervalle de temps étant choisi pour correspondre à une valeur de température et on analyse dans chaque intervalle la variation temporelle de la distance inter-image $d_2$ relative à chaque paire d'images consécutives de la pluralité d'image. On obtient une pluralité de courbes, dites courbes de décorrélation, de variation de la distance inter-image $d_2$ en fonction du temps (en secondes) pour une température donnée de la matière. Pour chaque courbe de décorrélation représentative de la variation de la distante inter-image $d_2$ dans un intervalle de temps donné, on détermine la valeur du temps caractéristique ($\tau_c$) de la variation de la distante inter-image $d_2$ sur cet intervalle de temps.

On peut, par exemple, déterminer le temps caractéristique ($\tau_c$) au moyen d'un ajustement (« fit ») d'une fonction exponentielle sur les données de la variation de la distance inter-image $d_2$ par rapport au temps et ceci sur chaque intervalle de temps. On obtient pour chaque intervalle de temps une valeur de temps caractéristique ($\tau_c$). On calcule aussi pour chaque intervalle de temps, l'inverse (1/$\tau_c$) du temps caractéristique ($\tau_c$), nommé « micro-dynamique » ($\mu$D, Hz). La grandeur « micro-dynamique » ($\mu$D, Hz) est représentative de la dynamique moléculaire de la matière non purement gazeuse soumise à une variation de température et hors équilibre thermodynamique. La courbe de variation de la « micro-dynamique » ($\mu$D, Hz) en fonction de la température mesurée de la matière non purement gazeuse analysée présente des pics hautement résolus permettant une détection de changements d'état physique de la matière non purement gazeuse -notamment de changements d'état physique de la matière non purement gazeuse à l'échelle nanométrique- non détectés par des méthodes d'analyse de matière non purement gazeuse à l'équilibre thermodynamique.

Aux fins de faciliter l'analyse, on calcule l'évolution micro-dynamique (« *micro-dynamics évolution* »), $\mu$DE(T) normalisée (%) correspondant à l'intégration par rapport à la température (T) de l'échantillon de la courbe de micro-dynamique ($\mu$D).

À partir de la courbe $\mu$DE(T), on définit des paramètres caractéristiques du changement d'état physique :

- $T_{10}$ : valeur de température en correspondance de laquelle la variation de la courbe représentative de l'évolution micro-dynamique ($\mu$DE(T) atteint la valeur de 10 % en début du changement d'état physique de la matière non purement gazeuse ;
- $T_{90}$ : valeur de température en correspondance de laquelle la variation de la courbe représentative de l'évolution micro-dynamique ($\mu$DE(T) atteint la valeur de 90 % en fin du changement d'état physique de la matière non purement gazeuse ;
- $T_{50}$ : valeur de température en correspondance de laquelle la variation de la courbe représentative de l'évolution micro-dynamique ($\mu$DE(T) atteint la valeur de 50 % et déterminée par régression linéaire entre toutes les valeurs mesurées d'évolution micro-dynamique ($\mu$DE(T) comprises entre $T_{10}$ et $T_{90}$ et représentative de la température de changement d'état physique, et ;
- $\Delta$T est représentatif de l'étendue de la plage de température dans laquelle se produit le changement d'état physique et évalué comme la valeur absolue de la différence des températures $T_{90}$ et $T_{10}$, $T_{90}$ correspondant à la température de fin de changement d'état physique et $T_{10}$ correspondant à la température de début de changement d'état physique. En particulier, $\Delta$T peut être représentatif de la multiplicité des formes cristallines présentes dans la matière non purement gazeuse analysée.

Le dispositif et le procédé selon l'invention permettent d'analyser un changement d'état physique d'une matière non purement gazeuse, en particulier en fin de procédé d'élaboration de ladite matière non purement gazeuse ou à toute étape du procédé d'élaboration de ladite matière non purement gazeuse, et cela sans nécessiter un prélèvement d'une fraction de ladite matière non purement gazeuse susceptible d'altérer cette analyse.

L'inventeur a observé qu'il est possible d'évaluer le risque de la survenue ultérieure du phénomène de blanchiment gras du chocolat en utilisant un dispositif et un procédé sur l'invention. Cette analyse représente un avantage concurrentiel considérable pour l'industrie chocolatière. En effet, le blanchiment gras du chocolat qui découle de la cristallisation incontrôlée de corps gras du chocolat constitue un défaut d'aspect d'un chocolat qui n'apparaît que plusieurs semaines après la fabrication du chocolat, le produit étant déjà proposé à la vente dans les magasins. Il en résulte une insatisfaction de la clientèle et par conséquence un manque à gagner.

L'inventeur a observé que l'analyse des températures de changement d'état physique de chocolat par la mise en oeuvre d'un procédé selon l'invention permet de distinguer, à la fin du procédé d'élaboration du chocolat ou à toute étape du procédé d'élaboration du chocolat, un chocolat susceptible de présenter ultérieurement le phénomène de blanchiment gras du chocolat d'un chocolat ne présentant pas ultérieurement ce phénomène.

À titre d'exemple non limitatif, l'inventeur a préparé un échantillon de chocolat correctement tempéré et un échantillon de chocolat incorrectement tempéré. Il a obtenu l'échantillon de chocolat correctement tempéré en soumettant une préparation de chocolat noir chauffé à la température de 50°C à une rampe de tempérage triphasique (50°C à 27°C, puis 27°C à 31°C, puis 31°C à 18°C). Il a obtenu l'échantillon de chocolat incorrectement tempéré en soumettent à une préparation du chocolat noir chauffé à la température de 50°C à une rampe de température monophasique (50°C à 18°C).

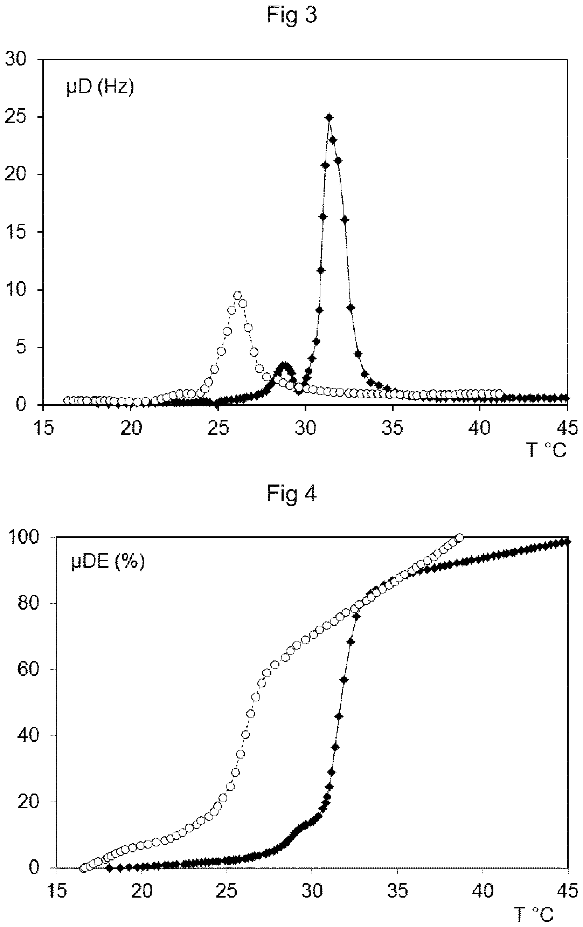

Il a soumis l'échantillon de chocolat correctement tempéré et l'échantillon de chocolat incorrectement tempéré à une analyse de changement d'état physique du chocolat en utilisant un dispositif et un procédé selon l'invention et une rampe de températures comprises entre 18°C et 45°C et une vitesse de variation de la température d'une valeur de 2°C/min . Les courbes de variation de la micro dynamique ($\mu$D) en fonction de la température pour ces 2 chocolats sont représentées en figure 3, dans laquelle la courbe identifiée par des ($\blacklozenge$) correspond au chocolat correctement tempéré et la courbe identifiée par des ($\bigcirc$) correspond au chocolat incorrectement tempéré. Les courbes d'évolution micro dynamique ($\mu$DE, %) en fonction de la température pour ces 2 chocolats sont représentées en figure 4, dans laquelle la courbe identifiée par des ($\blacklozenge$) correspond au chocolat correctement tempéré et la courbe identifiée par des (O) correspond au chocolat incorrectement tempéré. A partir des courbes d'évolution micro dynamique ($\mu$DE, %) en fonction de la température, ils ont déterminé les valeurs de $T_{10}$, $T_{50}$, $T_{90}$ et $\Delta T$ mesurées pour le chocolat correctement tempéré et pour le chocolat incorrectement tempéré. Les résultats sont présentés au tableau 1 ci-après.

Tableau 1

|  | Correctement tempéré | Incorrectement tempéré |
|---|---|---|
| $T_{10}$ | 28,7°C | 22,0°C |
| $T_{50}$ | 31,8°C | 28,0°C |
| $T_{90}$ | 36,7°C | 35,8°C |
| $\Delta T$ | 7,9°C | 13,8°C |

L'inventeur a observé qu'un chocolat présentant une température de début de changement d'état physique (transition cristalline ou transition polymorphique) de valeur sensiblement comprise entre 28°C et 33°C correspond à un chocolat présentant un risque réduit de blanchiment gras ultérieur, alors qu'un chocolat présentant une température de début de changement d'état physique de valeur sensiblement inférieure à 28°C, notamment de l'ordre de 26°C, correspond un chocolat présentant un fort risque de survenue ultérieure de blanchiment gras. La mesure de la température de changement d'état physique du chocolat réalisée au moyen d'un dispositif selon l'invention permet donc, par la mesure d'une température de changement d'état physique obtenue au moyen d'un dispositif et d'un procédé selon l'invention, d'évaluer et de prédire avec de bonnes chances de succès le risque de survenue du phénomène de blanchiment gras du chocolat au cours des phases d'élaboration du chocolat.

L'invention permet donc de prédire la survenue du phénomène macroscopique de blanchiment du chocolat par une analyse à l'échelle microscopique de changements d'état physique du chocolat.

L'inventeur a en outre observé que le simple prélèvement d'un micro échantillon de matière non purement gazeuse -et en particulier de chocolat- nécessaire pour son analyse par micro-DSC ne permet pas de réaliser une analyse directement sur le produit fini et ne permet pas de détecter de façon fiable un changement d'état physique de cette matière non purement gazeuse ni d'évaluer le risque de survenue du blanchiment gras dans le cas du chocolat.

Il va de soi que l'invention peut faire l'objet de nombreuses variantes de réalisation et applications. En particulier le dimensionnement et la conception du dispositif, notamment le choix de la source de rayonnement électromagnétique, du détecteur et leur configuration par rapport au porte échantillon peuvent varier sans sortir de la portée de protection des revendications.

**Revendications**

1. Dispositif (1) d'analyse d'un changement d'état physique d'une matière (3) non purement gazeuse, comprenant :

- une source d'un rayonnement électromagnétique cohérent (12), la source est disposée pour diriger le rayonnement électromagnétique cohérent sur une partie de surface libre de la matière et pour former un rayonnement électromagnétique diffusé par la matière;
- un dispositif de contrôle temporel de la température de la matière, adapté pour faire varier dans le temps la température de la matière ;
- un détecteur (13) d'au moins une grandeur d'au moins une partie de rayonnement électromagnétique diffusé par la matière ;
- des moyens de calcul d'une valeur représentative d'une vitesse de variation de ladite au moins une grandeur mesurée et représentative du changement d'état physique de la matière ;
- des moyens de mesure sans contact de la température (20) de ladite partie de la matière ;
- le dispositif d'analyse comprend un support de réception de la matière non purement gazeuse, adapté pour que cette matière portée par le support de réception présente au moins une partie de surface libre interposée entre la source et le support de réception, et **caractérisé en ce que** ;
- les moyens de mesure sans contact de la température étant positionnés de façon à mesurer la température en surface de ladite partie de surface libre de la matière non purement gazeuse .

2. Dispositif selon la revendication 1, **caractérisé en ce que** le détecteur est choisi dans le groupe formé d'un tube photomultiplicateur, d'une photodiode et d'un capteur matriciel.

3. Dispositif selon l'une des revendications 1 ou 2, **caractérisé en ce que** le dispositif de contrôle de la température de la matière est un dispositif à effet Peltier.

4. Dispositif selon l'une des revendications 1 à 3, **caractérisé en ce qu'**il comprend des moyens électroniques de programmation de la variation temporelle de température.

5. Dispositif selon l'une des revendications 1 à 4, **caractérisé en ce que** les moyens de mesure sans contact comprennent un capteur de rayonnement infrarouge.

6. Procédé d'analyse d'un changement d'état physique d'une matière (3) non purement gazeuse, dans lequel :

- on maintient au moins une partie de surface libre de la matière sous un rayonnement électromagnétique cohérent ;
- on fait varier dans le temps la température de la matière ;
- on réalise au cours de la variation de température une pluralité de mesures d'au moins une grandeur d'au moins une partie de rayonnement électromagnétique diffusé par la matière, les différentes mesures de la pluralité de mesures étant réalisées à des instants différents et pour des températures déterminées de la matière,
- - on mesure la température de ladite surface libre de la matière non purement gazeuse avec des moyens de mesure sans contact de la température (20) au cours de la variation temporelle de la température, et ;
- on déduit de la pluralité de mesures une valeur représentative d'une vitesse de variation de chaque grandeur mesurée et du changement d'état physique de la matière.

7. Procédé selon la revendication 6, **caractérisé en ce qu'**au moins une grandeur mesurée du rayonnement électromagnétique diffusé est une image d'au moins une partie d'un rayonnement électromagnétique diffusé.

8. Procédé selon l'une des revendications 6 ou 7, **caractérisé en ce qu'**au moins une grandeur mesurée du rayonnement électromagnétique diffusé est une image d'au moins une partie d'un rayonnement électromagnétique rétrodiffusé.

9. Procédé selon l'une des revendications 7 ou 8, **caractérisé en ce que** :

◦ on compare chaque image $I_n$ de la pluralité d'images mesurées avec une image $I_a$ antérieure de la pluralité d'images mesurées ;
◦ on calcule une valeur, dite distance inter-image $d_2$, représentative des différences que présente l'image $I_n$ en comparaison avec l'image $I_a$, puis ;
◦ on analyse l'évolution de ladite distance inter-image $d_2$ sur chaque intervalle de temps d'une pluralité d'intervalles de temps au cours desquels ont fait varier la température de la matière, et ;
◦ on déduit ladite valeur représentative de la vitesse de variation de la distance inter-image $d_2$ sur chaque intervalle de temps, ladite valeur étant représentative du changement d'état physique de la matière.

**10.** Procédé selon l'une des revendications 6 à 9, **caractérisé en ce qu'**on fait varier la température de la matière selon une rampe temporelle de température choisie dans le groupe formé des rampes de température croissante, des rampes de température décroissante, et des rampes de température alternativement croissante et décroissante.

**11.** Procédé selon l'une des revendications 6 à 10, **caractérisé en ce qu'**il est mis en oeuvre sur une chaîne d'élaboration de ladite matière non purement gazeuse.

**12.** Procédé selon l'une des revendications 6 à 11, **caractérisé en ce que** la matière non purement gazeuse est un corps gras, notamment du chocolat

**13.** Utilisation d'un dispositif (1) selon l'une des revendications 1 à 5, pour évaluer un risque de survenue d'un blanchiment de chocolat à toute étape de son élaboration.

**Patentansprüche**

**1.** Vorrichtung (1) zur Analyse einer Änderung des physikalischen Zustands eines nicht rein gasförmigen Stoffs (3), die enthält:

- eine Quelle einer kohärenten elektromagnetischen Strahlung (12), die Quelle ist angeordnet, um die kohärente elektromagnetische Strahlung auf einen freien Oberflächenteil des Stoffs zu richten und um eine vom Stoff gestreute elektromagnetische Strahlung zu formen;
- eine Vorrichtung zur zeitlichen Kontrolle der Temperatur des Stoffs, die geeignet ist, die Temperatur des Stoffs in der Zeit zu verändern;
- einen Detektor (13) mindestens einer Größe mindestens eines Teils der vom Stoff gestreuten elektromagnetischen Strahlung;
- Einrichtungen zur Berechnung eines Werts, der für eine Änderungsgeschwindigkeit der mindestens einen gemessenen Größe und für die Änderung des physikalischen Zustands des Stoffs repräsentativ ist;
- Einrichtungen zur kontaktlosen Messung der Temperatur (20) des Teils des Stoffs;
- die Analysevorrichtung enthält einen Aufnahmeträger des nicht rein gasförmigen Stoffs, der angepasst ist, damit dieser vom Aufnahmeträger getragene Stoff mindestens einen freien Oberflächenteil aufweist, der zwischen die Quelle und den Aufnahmeträger eingefügt ist, und

**dadurch gekennzeichnet, dass**:

- die Einrichtungen zur kontaktlosen Messung der Temperatur so positioniert sind, dass sie die Temperatur an der Oberfläche des freien Oberflächenteils des nicht rein gasförmigen Stoffs messen.

**2.** Vorrichtung nach Anspruch 1, **dadurch gekennzeichnet, dass** der Detektor aus der Gruppe ausgewählt wird, die von einer Photovervielfacherröhre, einer Photodiode und einem Matrix-Sensor gebildet wird.

**3.** Vorrichtung nach einem der Ansprüche 1 oder 2, **dadurch gekennzeichnet, dass** die Kontrollvorrichtung der Temperatur des Stoffs eine Vorrichtung mit Peltier-Effekt ist.

**4.** Vorrichtung nach einem der Ansprüche 1 bis 3, **dadurch gekennzeichnet, dass** sie elektronische Programmiereinrichtungen der zeitlichen Temperaturänderung enthält.

**5.** Vorrichtung nach einem der Ansprüche 1 bis 4, **dadurch gekennzeichnet, dass** die Einrichtungen zur kontaktlosen Messung einen Infrarotstrahlungssensor enthalten.

**6.** Verfahren zur Analyse einer Änderung des physikalischen Zustands eines nicht rein gasförmigen Stoffs (3), wobei:

- mindestens ein freier Oberflächenteil des Stoffs unter einer kohärenten elektromagnetischen Strahlung gehalten wird;
- die Temperatur des Stoffs zeitlich verändert wird;
- während der Temperaturänderung eine Vielzahl von Messungen mindestens einer Größe mindestens eines Teils einer vom Stoff gestreuten elektromagnetischen Strahlung durchgeführt wird, wobei die verschiedenen Messungen der Vielzahl von Messungen zu unterschiedlichen Zeitpunkten und für bestimmte Temperaturen des Stoffs durchgeführt werden,
- die Temperatur der freien Oberfläche des nicht rein gasförmigen Stoffs wird mit Einrichtungen zur kontaktlosen Messung der Temperatur (20) während der zeitlichen Änderung der Temperatur gemessen, und
- von der Vielzahl von Messungen wird ein für eine Änderungsgeschwindigkeit jeder gemessenen Größe und die Änderung des physikalischen Zustands des Stoffs repräsentativer Wert abgeleitet.

7. Verfahren nach Anspruch 6, **dadurch gekennzeichnet, dass** mindestens eine gemessene Größe der gestreuten elektromagnetischen Strahlung ein Bild mindestens eines Teils einer gestreuten elektromagnetischen Strahlung ist.

8. Verfahren nach einem der Ansprüche 6 oder 7, **dadurch gekennzeichnet, dass** mindestens eine gemessene Größe der gestreuten elektromagnetischen Strahlung ein Bild mindestens eines Teils einer rückgestreuten elektromagnetischen Strahlung ist.

9. Verfahren nach einem der Ansprüche 7 oder 8, **dadurch gekennzeichnet, dass**:

    o jedes Bild $I_n$ der Vielzahl von gemessenen Bildern mit einem früheren Bild $I_a$ der Vielzahl von gemessenen Bildern verglichen wird;
    o ein Wert, Zwischenbildabstand $d_2$ genannt, berechnet wird, der für Unterschiede repräsentativ ist, die das Bild $I_n$ im Vergleich mit dem Bild $I_a$ aufweist, dann
    ◦ die Entwicklung des Zwischenbildabstands $d_2$ in jedem Zeitintervall einer Vielzahl von Zeitintervallen analysiert wird, während denen die Temperatur des Stoffs geändert wird, und
    ◦ der für die Änderungsgeschwindigkeit des Zwischenbildabstands $d_2$ in jedem Zeitintervall repräsentative Wert abgeleitet wird, wobei der Wert für die Änderung des physikalischen Zustands des Stoffs repräsentativ ist.

10. Verfahren nach einem der Ansprüche 6 bis 9, **dadurch gekennzeichnet, dass** die Temperatur des Stoffs gemäß einer zeitlichen Temperaturrampe geändert wird, die aus der Gruppe ausgewählt wird, die von den Rampen steigender Temperatur, den Rampen sinkender Temperatur und den Rampen abwechselnd steigender und sinkender Temperatur gebildet wird.

11. Verfahren nach einem der Ansprüche 6 bis 10, **dadurch gekennzeichnet, dass** es in einer Erzeugungskette des nicht rein gasförmigen Stoffs durchgeführt wird.

12. Verfahren nach einem der Ansprüche 6 bis 11, **dadurch gekennzeichnet, dass** der nicht rein gasförmige Stoff ein Fettstoff ist, insbesondere Schokolade.

13. Verwendung einer Vorrichtung (1) nach einem der Ansprüche 1 bis 5, um die Gefahr des Auftretens eines Schokoladenreifs in jeder Stufe ihrer Erzeugung zu ermitteln.

**Claims**

1. Device (1) for analysing a change of physical state of a matter (3) that is not purely gaseous, comprising:

    - a source of a coherent electromagnetic radiation (12), the source is disposed to direct the coherent electromagnetic radiation onto a part of free surface of the matter and to form an electromagnetic radiation scattered by the matter;
    - a device for controlling the temperature of the matter over time, suitable for varying the temperature of the matter over time;
    - a detector (13) of at least one quantity of at least a part of electromagnetic radiation scattered by the matter;
    - means for calculating a value representative of a rate of variation of said at least one measured quantity and representative of the change of physical state of the matter;
    - means for contactlessly measuring the temperature (20) of said part of matter;
    - the analysis device comprises a support for receiving the matter that is not purely gaseous, suitable for this matter borne by the receiving support to have at least a part of free surface interposed between the source and the receiving support, and **characterized in that**
    - the contactless temperature measurement means are positioned so as to measure the temperature on the surface of said part of free surface of the matter that is not purely gaseous.

**2.** Device according to Claim 1, **characterized in that** the detector is chosen from the group formed by a photoelectric electron-multiplier tube, a photodiode and a matrix sensor.

**3.** Device according to one of Claims 1 and 2, **characterized in that** the device for controlling the temperature of the matter is a Peltier-effect device.

**4.** Device according to one of Claims 1 to 3, **characterized in that** it comprises electronic means for programming the temperature variation over time.

**5.** Device according to one of Claims 1 to 4, **characterized in that** the contactless measurement means comprise an infrared radiation sensor.

**6.** Method for analysing a change of physical state of a matter (3) that is not purely gaseous, wherein:

- at least a part of free surface of the matter is kept under a coherent electromagnetic radiation;
- the temperature of the matter is varied over time;
- during the temperature variation, a plurality of measurements are made of at least one quantity of at least a part of electromagnetic radiation scattered by the matter, the different measurements of the plurality of measurements being performed at different instants and for determined temperatures of the matter,
- the temperature of said free surface of the matter that is not purely gaseous is measured with contactless temperature measurement means (20) during the temperature variation over time, and;
- from the plurality of measurements, a value representative of a rate of variation of each measured quantity and of the change of physical state of the matter is deduced.

**7.** Method according to Claim 6, **characterized in that** at least one measured quantity of the scattered electromagnetic radiation is an image of at least a part of a scattered electromagnetic radiation.

**8.** Method according to one of Claims 6 and 7, **characterized in that** at least one measured quantity of the scattered electromagnetic radiation is an image of at least part of a backscattered electromagnetic radiation.

**9.** Method according to one of Claims 7 and 8, **characterized in that**:

O each image $I_n$ of the plurality of images is measured with a prior image $I_a$ of the plurality of measured images;
O a value, called inter-image distance value $d_2$, is calculated that is representative of the differences presented by the image $I_n$ in comparison with the image $I_a$, then;
O the changing of said inter-image distance $d_2$ on each time interval of a plurality of time intervals during which the temperature of the matter is made to vary is analysed, and;
O said value representative of the rate of variation of the inter-image distance $d_2$ on each time interval is deduced, said value being representative of the change of physical state of the matter.

**10.** Method according to one of Claims 6 to 9, **characterized in that** the temperature of the matter is made to vary according to a temperature ramp over time chosen from the group formed by increasing temperature ramps, decreasing temperature ramps, and alternately increasing and decreasing temperature ramps.

**11.** Method according to one of Claims 6 to 10, **characterized in that** it is implemented on a line for developing said matter that is not purely gaseous.

**12.** Method according to one of Claims 6 to 11, **characterized in that** the matter that is not purely gaseous is a fatty body, notably chocolate.

**13.** Use of a device (1) according to one of Claims 1 to 5, for assessing the risk of occurrence of bleaching of chocolate at any step in its development.

Fig 1

Fig 2

## Fig 3

## Fig 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20050037499 A1

**Littérature non-brevet citée dans la description**

- **ELIOT et al.** *Food Hydrocolloïds,* 2005, vol. 19, 279-287